# EUROPEAN PATENT APPLICATION

(11) **EP 1 818 024 A1**
(43) Date of publication of application: **15.08.2007**
(21) Application number: 06101439.5
(22) Date of filing: 09.02.2006
(51) Int. Cl.: A61F 2/44

(54) **Plastically deformable intervertebral fusion implant**

(71) Applicant: Inion Oy, 33520 Tampere (FI)
(72) Inventor: PÖSEL, Andreas, 34130, Ylinen (FI)
(74) Representative: Kaukonen, Juha Veikko

(57) **Abstract**

An implant to be inserted in the disk space between a lower vertebra and an upper vertebra. The implant comprises a body (2) having a lower surface (5) and an upper surface (6), and an aperture (7) extending through the body (2) in the direction of the height of the body (2) from a lower side (8) of the body to an upper side (9) of the body. The body (2) is arranged to reduce its height permanently under the load the body (2) is exposed to between a lower vertebra and an upper vertebra.

## Description

### FIELD OF THE INVENTION

The invention relates to an implant to be inserted in the disk space between a lower vertebra and an upper vertebra, the implant comprising a body having a lower surface and an upper surface, and an aperture extending through the body in the direction of the height of the body from a lower side of the body to an upper side of the body.

### BACKGROUND OF THE INVENTION

Spinal cages or interbody fusion devices are frequently used to facilitate bony fusion between two vertebral bodies of the human spine. Interbody fusion of the spine is a surgical treatment procedure which is applied to patients with chronic back pain due to degenerative disk disease (DDD), if it cannot be cured with non-surgical treatment. In DDD the intervertebral disk, a flexible shock-absorbing element, is bulged, herniated, or ruptured due to aging or trauma. Typically the disk bulges out and impinges on the spinal cord that runs through the vertebrae all along the spine in the spinal canal.

The aim of the interbody fusion procedure and the purpose of using spinal cages is to restore the original disk height between the vertebral bodies and to permanently immobilize the two vertebrae in order to alleviate the patient's pain. Some examples of known spinal cages are introduced in documents WO 99/08627, EP 1 138 285, and US 5,749,916.

In the surgical procedure, the affected intervertebral disk is excised and a spinal cage is filled with bone graft and slid or impacted into the intervertebral space. The spinal cages can be made of biodegradable or biostable materials. Biostable spinal cages are usually made of metal, but some biostable implants are made of polymers, such as PEEK (poly-ether-etherketone). In typically 3 to 6 months after surgery, bony fusion occurs between the two adjacent vertebrae when the bone graft turns into hard bone and fuses with the vertebral bodies. In a percentage of patients, no solid fusion is obtained, which in some cases is attributed to the fact that cages are too rigid constructs. Bone needs a pressure stimulus to heal and that pressure is "shielded" off from the bone graft by too rigid a cage. This effect is commonly known as "stress shielding", and the use of spinal cages is declined partly due to this disadvantage.

### BRIEF DESCRIPTION OF THE INVENTION

An object of the present invention is to provide an implant to alleviate the above disadvantages.

The surgical implant of the invention is characterized in that the body is arranged to reduce its height permanently under the load the body is exposed to between a lower vertebra and an upper vertebra.

An idea of the invention is that the spinal cage according to the invention is capable of restoring the intervertebral disc height but at the same time has the ability to share load with the graft inside the spinal cage through a controlled adaptation or reduction in spinal cage height. It should be noted that the spinal cage is hereafter on referred to as 'cage'.

The cage possesses design features or, alternatively, material features, which allow it to reduce its height permanently within limits when defined load acting along the axis of the spine is exceeded. The cage would then give in to the force until the bone graft inside the cage receives part of the load. The cage stops giving in to the external load as soon as the graft is compacted enough so that it can take over some of the load. The cage follows the so-called subsidence (settling) of the graft. An advantage of the cage of the invention is that it may accelerate the process of bony fusion as the graft material is compressed within the cage and, therefore, it may increase the percentage of successful fusions. It is to be noted that the deformation of the cage is permanent. The term 'permanent' means here permanent plastic deformation. In other words, if the load is taken away again, the cage will not go back to its original shape. It will stay compressed. The cage does not show any significant elastic behavior.

Further, the idea of an embodiment of the invention is that the cage is made of metal or a metal alloy, for example titanium and its alloys, stainless steel, tantalum, niobium, and magnesium and its alloys. An advantage is that high compression strength at relatively small wall thicknesses is achieved, leaving a big internal opening in the body of the cage to take the graft.

Further, the idea of an embodiment of the invention is that the cage is made of a resorbable material, like polymer, copolymer, or polymer mixtures, i.e. the cage will degrade over time by the action of enzymes, by hydrolytic action, and/or by other similar mechanisms in the human body, or it will erode or degrade over time due, at least in part, to contact with substances found in the surrounding tissue fluids, cellular action, and the like, or it will be broken down and absorbed within the human body, for example, by a cell, a tissue, and the like. An advantage is that an increasing amount of the load resulting from the body's weight and the movements in the spine is transferred to the graft as the cage resorbs and loses its strength. A second advantage is that the cage ultimately completely disappears from the human body and cannot cause any late complications, such as cage migration, screw backout, metal induced allergic reactions, or corrosion problems. A third advantage is that when a revision surgery is undertaken, the resorbable cage does not have to be removed but new hardware can be inserted through the remnants of the cage should there be any. A fourth advantage is a better assessment of the fusion result through absence of artifacts when taking radiographs or MRI pictures. A fifth advantage is that better inherent elasticity in the material potentially leads to less stress shielding per se as the polymer's modulus of elasticity is closer to that of bone than that of metal.

### BRIEF DESCRIPTION OF THE DRAWINGS

In the following, the invention will be described in greater detail by means of preferred embodiments and with reference to the accompanying drawings, in which
Figure 1 is a schematic perspective view of a spinal cage according to the invention,
Figure 2a is a schematic side view of the the spinal cage shown in Figure 1 prior to its deformation between a lower vertebra and an upper vertebra,
Figure 2b is a schematic side view of the spinal cage shown in Figure 1 after its deformation between a lower vertebra and an upper vertebra,
Figure 3a is a schematic side view of a second spinal cage according to the invention and prior to its deformation between a lower vertebra and an upper vertebra,
Figure 3b is a schematic side view of the spinal cage shown in Figure 3a after its deformation between a lower vertebra and an upper vertebra,
Figure 4a is a schematic side view of a third spinal cage according to the invention and prior to its deformation between a lower vertebra and an upper vertebra,
Figure 4b is a schematic side view of the spinal cage shown in Figure 4a after its deformation between a lower vertebra and an upper vertebra,
Figures 5a to 5c are schematic views of a fourth spinal cage according to the invention,
Figures 6a to 6c are schematic views of a fifth spinal cage according to the invention,
Figures 7a to 7b are schematic views of a sixth spinal cage according to the invention,
Figures 8a to 8b are schematic views of a seventh spinal cage according to the invention,
Figures 9a to 9b are schematic views of an eighth spinal cage according to the invention, and
Figures 10a to 10b are schematic views of a ninth spinal cage according to the invention.

For the sake of clarity, the figures show the invention in a simplified manner. Like reference numerals identify like elements.

### DETAILED DESCRIPTION OF CERTAIN PREFERRED EMBODIMENTS OF THE INVENTION

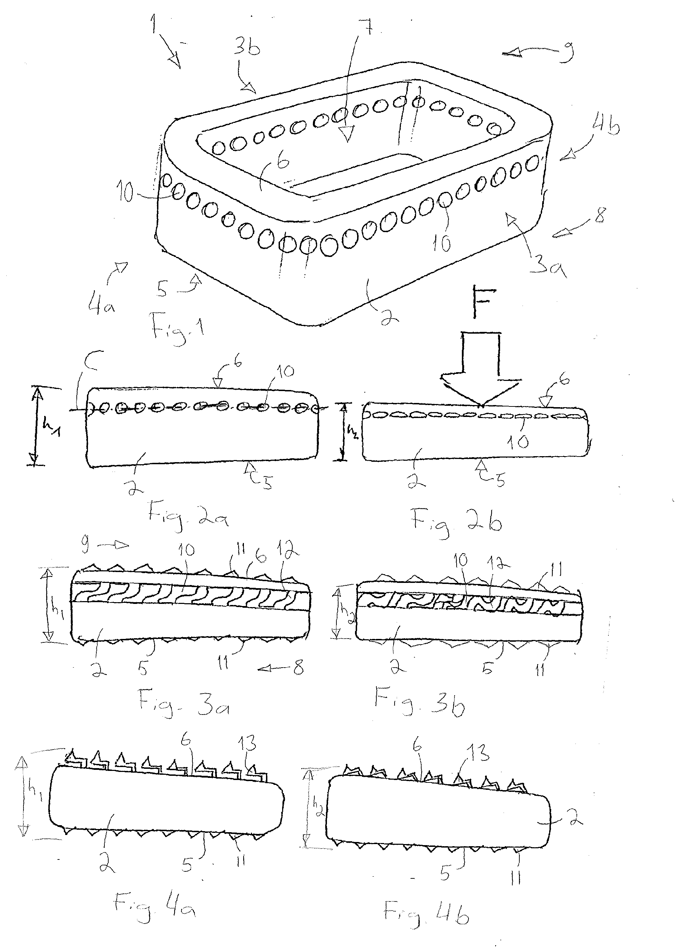

Figure 1 is a schematic perspective view of a spinal cage according to the invention. The cage 1 is intended to be inserted between adjacent vertebrae. The cage 1 is particularly useful in an anterior approach for spinal fusion. Nevertheless, other surgical approaches may be utilized including posterior, anterior-lateral, posterior-lateral, etc. Cage 1 could be used in all segments of the spine, i.e. cervical, thoracic and lumbar.

The cage 1 has a body 2 that can be manufactured from a resorbable material, such as resorbable polymer, copolymer, or polymer mixtures. Examples of suitable polymers are polymers based on polylactide (PLA), polyglycolide (PGA), and trimethylenecarbonate (TMC). As used herein, the term 'resorbable material' means that the material is biodegradable, bioerodible, and/or bioabsorbable.

The body 2 can also be manufactured from a bio-stabile polymer, copolymer or polymer mixtures, or from metal or metal alloy. The term 'bio-stabile material' means all materials not belonging to resorbable materials. Suitable bio-stabile polymers comprise, for instance, polyaryletherketones (PAEK), such as polyetheretherketone (PEEK), polyamides, and polyolefines, such as ultra-high-molecular-weight-high-density-polyethylene (UHMWHDPE) or linear-low-density-polyethylene (LLDPE). Suitable metals or metal alloys comprise titanium and its alloys, stainless steel, tantalum, niobium, and magnesium and its alloys, gold and silver.

The body 2 has a frame-like shape, when seen from the top side of the cage 1, the body 2 including first and second side walls 3a, 3b and first and second end walls 4a, 4b. The shape of the body 2 can also be a circle, an oval, kidney shaped, a trapezoid, a cylinder, or an ellipse, for instance.

In this embodiment shown in Figure 1, the first end wall 4a is somewhat higher that the second end wall 4b. Side walls 3a, 3b are tapered towards the second end wall 4b. Furthermore, walls 3a, 3b, 4a, 4b have substantially planar outer and inner surfaces in the embodiment shown in Figure 1. Alternatively, at least some of them can be non-planar, they can make a curve inwards or outwards, etc.

The body includes a lower surface 5 and an upper surface 6. Both the lower and the upper surface 5, 6 are generally planar and smooth, but, of course, they can be designed in some other way, too. For example, the surfaces can be curved towards the upper side 9 or lower side 8 of the cage, or they can be curved around an axis parallel to the longitudinal direction of said wall.

The lower surface 5 is intended to be arranged against a lower vertebra, whereas the upper surface 6 is intended to be arranged against an upper vertebra. It is to be noted that vertebrae are not shown in the figures. The lower surface 5 and/or the upper surface 6 can also have ridges, serrations, spikes, or some other protrusions that are intended to archieve the securing of the cage 1 to the adjacent vertebra.

The side walls 3a, 3b and the end walls 4a, 4b define an aperture or hole 7 that extends through the body from the lower side 8 of the body 2 to the upper side 9 of the body 2. It is to be noted that the cage 1 can also include two, three, or even more apertures 7. The aperture 7 is filled with bone graft material before the cage 1 is inserted in its place between the vertebrae. The use of the bone graft is known as such, and therefore it is not discussed here in detail. It should be noted, that the bone graft material can be autologous or autograft, allograft, xenograft, or synthetic bone graft. The cage 1 can have one or more bars or a net-like structure at one end of the aperture 7. The purpose of these structures is to prevent the bone graft material from slipping away from the aperture 7 during operational stages.

The walls of the body include a plurality of holes 10 that extend from the outer surface of the body all the way through to the aperture 7. The holes 10 reduce the cross-sectional area of the body 2 in a section of plane perpendicular to the height of the body 2.

Hole 10 can also be a blind hole, and its cross-section can be not only a circle but also a polygon, ellipse, or any other shape. The cross-sectional areas of the holes 10 are the same in all the holes 10, but this is not an essential feature. Multiple rows of holes are also possible. However, it should be noted here that some embodiments of the cage according to the invention do not include holes 10 at all.

The function of the cage 1 is discussed in connection with Figures 2a and 2b.

Figure 2a is a schematic side view of the cage shown in Figure 1 prior to its deformation between a lower vertebra and an upper vertebra, and Figure 2b is a schematic side view of the same cage after its deformation between said vertebrae.

The cage 1 is designed so that its height decreases when load or stress acting through the upper vertebra exceeds a limit value. In Figure 2a the body 2 has its original height h₁. In other words, the body 2 is in its uncompressed state in Figure 2a. The body 2 is in this uncompressed form while the aperture 7 is filled with bone graft, and prior to the insertion of the cage 1 in its place between the lower and upper vertebrae. It is also possible that, instead of one cage 1, two or more cages 1 can be inserted between the same vertebrae.

The insertion procedure of the cage 1 according to the invention into an intervertebral space between adjacent vertebrae employs method steps and equipment known in the art as such. Therefore, the procedure is not discussed in detail in this description. The intervertebral space can be enlarged by means of a suitable tool and the bone graft is compressed in the aperture 7 of the cage. The aperture 7 is filled with graft material in order to achieve optimal pre-compression of the graft material when the cage is inserted between the vertebral endplates.

In Figure 2b the body 2 is shown in its compressed state. Its height is permanently decreased within predefined limits from h₁ to h₂. Typically this means permanent reduction of height by an amount of 0.2 to 2 mm.

The decrease in height is due to force F that is directed to the body 2 from the upper vertebra. The body 2 is exposed to the force F as soon as the cage 1 is placed between vertebrae. The force F is transferred through the cage 1 by the body's weight above the affected segment of the spine or by flexion, extension, or lateral bending.

The body 2 then gives in to the force F until the bone graft inside the cage receives part of the load. The body 2 stops giving in to the external load as soon as the bone graft in the aperture 7 is compacted enough so that it can take over some of the load falling from the upper vertebra to the cage. Therefore, the body 2 shares the force F with the bone graft, and the height decrease of the cage 2 follows the so-called subsidence (settling) of the graft bone. This means that the graft block or compacted graft material becomes "shorter" either by high degree of compaction, by partial graft resorption during the advancement of fusion, or by sinking of the graft into the vertebral bone. The phenomena mentioned last takes place especially when the vertebral endplate is shaped or decorticated before the placement of the cage 1.

The force F can be in the range of 10 to 1000 N depending on the part or section of the spine where the cage 1 is arranged and the weight of the patient. The compacted bone graft in the aperture 7 together with the compressed body 2 has sufficient strength to maintain the adjacent vertebrae in a desired spatial relation during healing and fusion.

The above mentioned compression or height reduction feature can be achieved in both metallic cages and polymeric cages. In metallic cages, the reduction of body height can be based on plastic deformation of the metallic material. The body 2 may deform plastically under load immediately after the yield strength of the material of the body 2 is exceeded. In the cage 1 shown in Figures 1 to 2b, the yield strength of the body 2 is first exceeded in a section of plane C perpendicular to the height of the body 2 and coinciding with the centre axis of the holes 10. This is because the cross-sectional area of the body 2 is at its minimum in said section of plane C, and stress caused by the force F reaches its maximum in said section of plane C. The yield stress of the metal material of the cage 1 can be set on a desired level not only by the selection of the metal or metal alloy but also by suitable heat treatment of the material.

Cages 1 made of suitable polymers can also deform plastically in the same way as the metallic ones discussed above. Examples of such polymers include PEEK, or even PLA and PGA polymers.

Alternatively, viscoelastic properties of polymers can be utilized in the deformation of cages 1. It is well-known that polymers show creep, i.e. they deform if they are held under a continuous load for a time. This results from the viscoelastic flow of the polymer with time. In this case no permanent plastic deformation is achieved, if a defined load is applied only for a short time to the cage 1. However, plastic deformation will occur, if the load is held for a longer period of time, for example 1 hour, or 12 hours, or more. The viscoelastic properties of the polymer the cage 1 is made of then lead to a deformation of the cage 1 through rearrangement of the molecular chains within the polymer when set under continuous load at body temperature. In cages 1 made of polymer materials, permanent deformation can occur under continuous load over a duration of one to several hours or days.

It is to be noted that the height decrease is not essentially equal in all parts of the cage 1, although an equal decrease takes place in the figures. In this way, the cage 1 can be adapted to the relief topology of the surface of the vertebral end plates.

Figure 3a is a schematic side view of a second cage according to the invention and prior to its deformation between a lower vertebra and an upper vertebra, and Figure 3b is a schematic side view of the same cage after its permanent deformation between a lower vertebra and an upper vertebra.

The body 2 of the cage 1 comprises a plurality of holes 10, the cross-section of which resembles the letter S. The holes 10 extend from the outer surface of the body 2 to the aperture 7, the aperture 7 being not shown in Figures 3a and 3b.

Between the holes 10, there are arranged relatively thin bridges 12 that connect the lower and upper portions of the body 2. The bridges 12 undergo permanent deformation by bending under the load applied to the cage 2 arranged between two vertebrae, the deformation inducing the reduction of height of the cage 1.

The design of the cage shown in Figures 3a, 3b is especially useful if the body 2 is made of metal or metal alloy. Of course, the cage of Figures 3a, 3b can be made of polymer materials, too.

The holes 10 can also be shaped in some other way to produce bendable bridges 12 between them.

The lower surface 5 and upper surface 6 include projections 11. The purpose of the projections 11 is to help to anchor the cage 1 to the vertebrae adjacent to the cage 1. The projections 11 can be ridges, barbs or pegs, for instance. It should be noted that the projections 11 are optional elements of the cage 1.

Figure 4a is a schematic side view of a third cage according to the invention and prior to its deformation between a lower vertebra and an upper vertebra, and Figure 4b is a schematic side view of the cage after its deformation between a lower vertebra and an upper vertebra.

The body 2 of the cage 1 comprises deformable projections 13 on its upper surface 6. The projections 13 deform permanently under a specific load caused by the upper vertebra. The mechanism of the permanent deformation can be plastic deformation or creep, depending on the manufacturing material of the deformable projections 13.

The deformable projections 13 can have a sharp edge or tip that is intended to help with attaching the cage 1 to the upper vertebra.

Additionally, the lower surface 5 includes projections 11 for keeping the cage 1 immobile with respect to the lower vertebra. The projections 11 are only optional, i.e. the cage 1 can also be realized without them.

Alternatively, the deformable projections 13 can be arranged to the lower surface 5, or both the upper 6 and lower 5 surfaces of the body 2.

Figures 5a to 5c are schematic views of a fourth spinal cage according to the invention. The cage 1 has a body 2 including a groove 14. The groove 14 can circulate completely around the lower part of the body 2. Furthermore, the gage 1 includes a bevelled surface 15 on the inner surface of the lower part of the body 2. The cage 1 is in its non-compressed state in Figure 5b. In Figure 5c, the cage 1 is in its compressed state in which its height is decreased permanently due to a force directed to the body 2 from the upper vertebra. The body is at least mainly deformed in a bending area that is situated between the groove 14 and the bevelled surface 15.

Figures 6a to 6c are schematic views of a fifth spinal cage according to the invention. Here the cage 1 comprises a plurality of slots 16 that are arranged alternately on the outer and the inner surface of the body 2. The slots 16 can circumvent the whole body 2. Permanent deformation of the cage 1 takes place at least mainly by the bending of thin bridges between the slots 16. The cage 1 is in its non-compressed state in Figure 6b, and in its compressed state in Figure 6c.

Figures 7a to 7b are schematic views of a sixth spinal cage according to the invention. The cage 1 is in its non-compressed state in Figure 7a and in its compressed state in Figure 7b. The cage 1 comprises a body 2 including a first body part 18 and a second body part 19. The first body part 18 is at a distance from the second body part 19 in a non-compressed cage, as shown in Figure 7a. The body parts 18, 19 are connected to each other by a bending element 22. The bending element 22 resembles a flange or collar that frames the cage 1. Permanent deformation of the cage 1 takes place at least mainly by the bending of the bending element 22. It is not necessary that the bending element 22 circumvent the cage 1 in a continuous manner; instead, the bending element 22 can be formed of two or more discrete sub-elements that are arranged at a distance from each other.

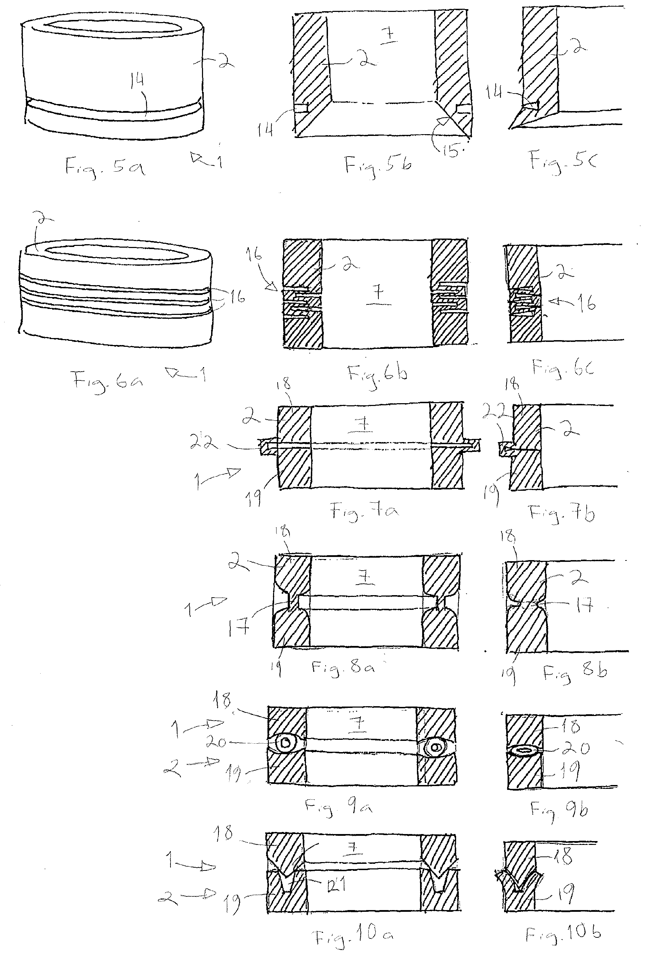

Figures 8a to 8b are schematic views of a seventh spinal cage according to the invention. The cage 1 is in its non-compressed state in Figure 8a and in its compressed state in Figure 8b. The cage 1 comprises a body 2 including a first body part 18 and a second body part 19, the body parts 18, 19 being connected to each other by a compression element 17. The compression element 17 is arranged to compress under a force the body 2 is exposed to between vertebrae.

It is not essential that the complete body 2 is made of the same material. The body 2 can include one or more sections that permanently decrease its height under load, whereas other sections of the body 2 do not behave this way. Said other sections can, for example, behave elastically, i.e. they will recover their original shape like a spring, or they can be of a very tough material that does not deform at all under stresses the cage 1 is exposed to in an intervertebral space. Figures 9a to 9b are schematic views of an eighth spinal cage according to the invention. The cage 1 comprises a first body part 18, a second body part 19, and a third body part 20. The third body part 20 has a tube-like cross-section and is made of a different material than the first and second 18, 19 body parts. The third body part 20 can be made of, for instance, a resorbable material that resorbs fast in the human body. Due to the resorbing, the third body part 20 loses its strength as the height of the body 2 decreases permanently between vertebrae. The first and second body parts 18, 19 can also be made of a resorbable material but, alternatively, one of them or both of them can be made of bio-stabile material. It is possible, of course, that the third body part 20 is manufactured of a bio-stabile material, in which case the third body part 20 deforms plastically under a force the body 2 is exposed to between vertebrae.

The cross-section of the third body part 20 can also be solid and its periphery can be round, oval, polygon, etc.

Figures 10a to 10b are schematic views of a ninth spinal cage according to the invention. The cage 1 has a two-part body 2 comprising a first body part 18 and a second body part 19. The surface of the first body part 18 contacting with the second body part 19 is tapered towards the second body part 19, whereas the surface of the second body part 19 contacting the first body part 18 includes a channel 21 or groove. The tapered first body part 18 penetrates into the channel 21 under the load between vertebrae, decreasing the height of the cage 1.

It will be obvious to a person skilled in the art that as technology advances, the inventive concept can be implemented in various ways. The invention and its embodiments are not limited to the examples described above but may vary within the scope of the claims.

## Claims

1. An implant to be inserted in the disk space between a lower vertebra and an upper vertebra, the implant comprising
a body (2), having a lower surface (5) and an upper surface (6), and
an aperture (7) extending through the body (2) in the direction of the height of the body (2) from a lower side (8) of the body to an upper side (9) of the body, **characterized in that**
the body (2) is arranged to reduce its height permanently under the load the body (2) is exposed to between a lower vertebra and an upper vertebra.

2. An implant as claimed in claim 1**, characterized in that** the body (2) is made of resorbable material.

3. A surgical implant as claimed in claim 2**, characterized in that** the resorbable material comprises a polymer or copolymer or polymer mixture.

4. A surgical implant as claimed in claim 1, **characterized in that** the body (2) is made of bio-stabile material.

5. A surgical implant as claimed in claim 4, **characterized in that** the bio-stabile material comprises a polymer or a copolymer or a mixtures thereof.

6. A surgical implant as claimed in claim 4, **characterized in that** the bio-stabile material comprises a metal or a metal alloy.

7. A surgical implant as claimed in any one of claims 1 to 6, **characterized in that** the reduction of height takes place by plastic deformation of the material of the body (2).

8. A surgical implant as claimed in any one of claims 1 to 5, **characterized in that** the reduction of height takes place by the creep of the material of the body (2).

9. A surgical implant claimed in any one of claims 1 to 8, **characterized in that** the body (2) has holes (10) that are arranged to reduce the cross-sectional area of the body (2) in a section of plate (C) perpendicular to the height of the body (2).

10. A surgical implant as claimed in claim 9, **characterized in that** at least one of the holes (10) extends from outside the body (2) to the aperture (7).

11. A surgical implant as claimed in claim 9 or 10, **characterized in that** the holes (10) have a round cross-section.

12. A surgical implant as claimed in claim 9 or 10, **characterized in that** the holes (10) have a cross-section that is shaped like the letter S.

13. A surgical implant as claimed in any one of claims 1 to 12, **characterized in that** at least the lower surface (5) or the upper surface (6) of the body comprises one or more projections (13) that are arranged to reduce their height permanently under said load.

14. A surgical implant as claimed in claim 13, **characterized in that** the projection (13) has a sharp edge that is arranged to anchor the implant to bone.

15. A surgical implant as claimed in any one of claims 1 to 14, **characterized in that** the body (2) is designed to deform permanently under a load that is in the range of 10 to 1000 N.

16. A surgical implant as claimed in any one of claims 1 to 15, **characterized in that** the height of the body (2) is designed to decrease permanently 0.2 to 2 mm.

17. A surgical implant as claimed in claim 8, **characterized in that** the reduction of height takes place after a continuous load over a duration of one hour to several days.

18. A surgical implant as claimed in any one of claims 1 to 17, **characterized in that** the aperture (7) is filled with bone graft.

19. A surgical implant as claimed in any one of claims 1 to 18, **characterized in that** the cage (1) is made of at least two different materials.
